(19) Europäisches Patentamt
European Patent Office
Office européen des brevets

(11) EP 0 821 946 B1

(12) EUROPÄISCHE PATENTSCHRIFT

(45) Veröffentlichungstag und Bekanntmachung des Hinweises auf die Patenterteilung:
**05.03.2003 Patentblatt 2003/10**

(51) Int Cl.⁷: **A61K 7/42**

(21) Anmeldenummer: **97112202.3**

(22) Anmeldetag: **17.07.1997**

# (54) Stabile Lichtschutzzubereitungen mit einem Gehalt an grenzflächenaktiven Glucosederivaten und wasserlöslichen UV-Filtersubstanzen

Stable sunscreening compositions containing surface-active glucose derivatives and water-soluble UV-filters

Compositions stables de protection solaire contenant des dérivés tensio-actifs de glucose et des filtres ultraviolets hydrosolubles

(84) Benannte Vertragsstaaten:
**AT BE CH DE ES FR GB IT LI NL SE**

(30) Priorität: **02.08.1996 DE 19631222**

(43) Veröffentlichungstag der Anmeldung:
**04.02.1998 Patentblatt 1998/06**

(73) Patentinhaber: **Beiersdorf Aktiengesellschaft
20245 Hamburg (DE)**

(72) Erfinder:
- **Gers-Barlag, Heinrich, Dr.
25495 Kummerfeld (DE)**
- **Müller, Anja
20253 Hamburg (DE)**

(56) Entgegenhaltungen:

| | |
|---|---|
| **EP-A- 0 647 443** | **WO-A-96/14053** |
| **DE-A- 4 412 081** | **FR-A- 2 698 004** |
| **US-A- 5 037 901** | **US-A- 5 322 683** |

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 821 946 B1

## Beschreibung

Die vorliegende Erfindung betrifft kosmetische und dermatologische Lichtschutzzubereitungen, insbesondere hautpflegende kosmetische und dermatologische Lichtschutzzubereitungen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutze gegen UVB-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubstanzen zur Verfügung zu haben, da auch dessen Strahlen Schäden hervorrufen können. So ist erwiesen, daß UVA-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, z.B. Hydroxylradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Die 2-Phenylbenzimidazol-5-sulfonsäure bzw. ihre Salze, insbesondere das Natrium-, das Kalium und das TEA-Salz, beispielsweise erhältlich unter der Bezeichnung Eusolex® 232 der Merck AG, welches sich durch folgende Strukturformel auszeichnet:

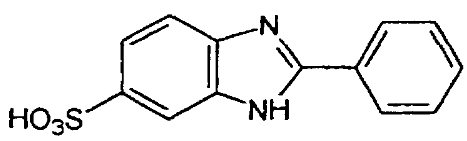

ist eine an sich vorteilhafte, in Wasser lösliche UV-Filtersubstanz. Der Hauptnachteil dieser Substanz ist an sich, daß der Elektrolytcharakter dieser Verbindung eine elegante und stabile kosmetische Formulierung erschwert. Für andere wasserlösliche, insbesondere sulfonierte UV-Filtersubstanzen gilt oft Vergleichbares.

Eine weitere bekannte und vorteilhafte, allerdings wasserunlösliche, Lichtschutzfiltersubstanz ist das 4-(tert. -Butyl)-4'-methoxydibenzoylmethan, welches sich durch die Struktur

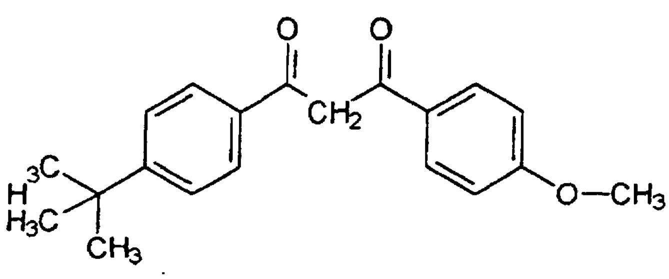

auszeichnet, und von Givaudan unter der Marke Parsol® 1789 verkauft wird

Der Hauptnachteil dieser Substanz ist eine gewisse Instabilität gegenüber UV-Strahlung, so daß es zweckmäßig ist, Zubereitungen mit einem Gehalt an dieser Substanz auch gewisse UV-Stabilisatoren einzuverleiben.
Ein weiterer vorteilhafter UVB-Filter ist der 4,4',4"-(1,3,5-Triazin-2,4,6-triyltriimino)-tris-benzoësäure-tris (2-ethylhexylester), synonym: 2,4,6-Tris-[anilino-(p-carbo-2'ethyl-1'-hexyloxy)]-1,3,5-triazin.

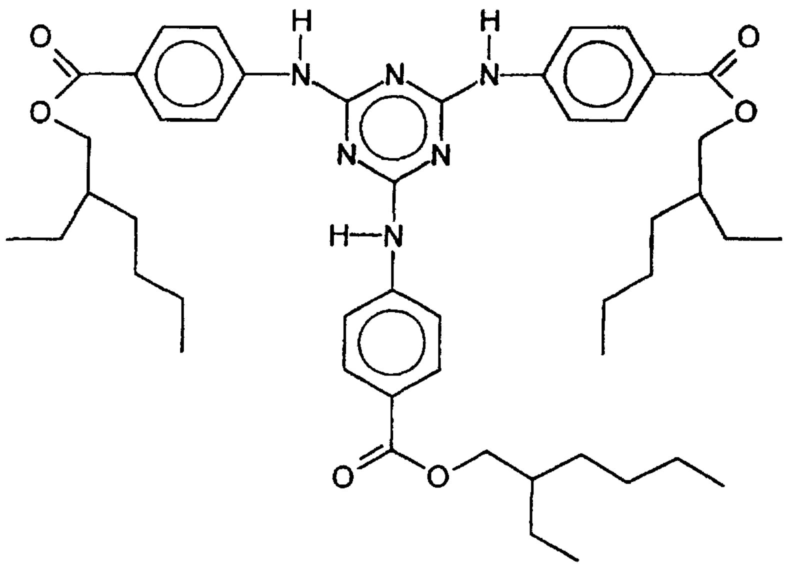

Diese UVB-Filtersubstanz wird von der BASF Aktiengesellschaft unter der Warenbezeichnung UVINUL® T 150 vertrieben und zeichnet sich durch gute UV-Absorptionseigenschaften aus.
Der Hauptnachteil dieses UVB-Filters ist die schlechte Löslichkeit in Lipiden. Bekannte Lösungsmittel für diesen UVB-Filter können maximal ca. 15 Gew.-% dieses Filters lösen, entsprechend etwa 1 - 1,5 Gew.-% gelöster, und damit aktiver, UV-Filtersubstanz.
Eine weitere vorteilhafte Lichtschutzfiltersubstanz ist der 4-Methylbenzylidencampher, welcher sich durch die Struktur

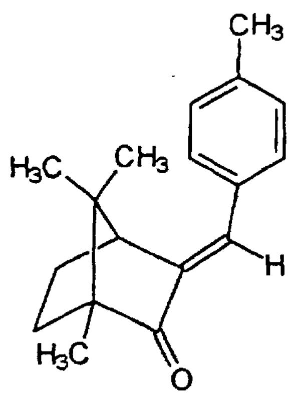

auszeichnet und von Merck unter der Marke Eusolex® 6300 verkauft wird. Diese Substanzen zeichnen sich an sich durch gute UV-Filtereigenschaften aus. Gerade in Kombination miteinander oder anderen als Festkörper vorliegenden Substanzen ist ihre Einsatzkonzentration jedoch begrenzt.
Eine weiterere vorteilhafte Lichtschutzfiltersubstanz ist das 2-Ethylhexyl-p-methoxy-cinnamat, welches von Givaudan unter der Bezeichnung Parsol® MCX erhältlich ist und sich durch folgende Struktur auszeichnet:

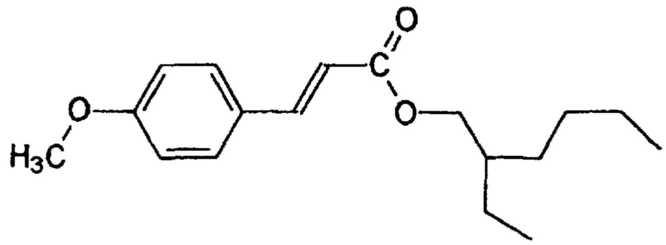

Noch eine weiterere vorteilhafte Lichtschutzfiltersubstanz ist das Ethylhexyl-2-cyano-3,3-diphenylacrylat (Octocrylen), welches von BASF unter der Bezeichnung UVINUL® N 539 erhältlich ist und sich durch folgende Struktur auszeichnet:

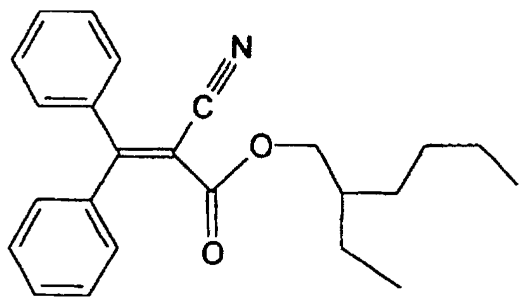

Insbesondere wenn mehrere der unter Normalbedingungen kristallin vorliegenden Lichtschutzsubstanzen vorliegen, beispielsweise gewählt aus der Gruppe 4,4',4"-(1,3,5-Triazin-2,4,6-triyltriimino)-tris-benzoesäure-tris (2-ethylhexylester), 4-Methylbenzylidencampher, 4-(tert.-Butyl)-4'-methoxydibenzoylmethan und Titandioxid, sind gemäß den Lehren des Standes der Technik nur jeweils geringe Einsatzkonzentrationen und damit niedrige Lichtschutzfaktoren möglich, es sei denn, der Anteil der Ölphase würde überproportional erhöht, was aber ebenfalls Nachteile hätte.

UV-Absorber bzw. UV-Reflektoren sind die meisten anorganischen Pigmente, die bekannterweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen.

Die anorganischen Pigmente zeichnen sich an sich durch gute Lichtschutzwirkung aus. Sie haben jedoch den Nachteil, daß es schwierig ist, sie in befriedigender Weise solchen Formulierungen einzuverleiben. Nur wenn die Partikel in der endgültigen Formulierung sehr klein sind, werden sie nach dem Auftragen auf die Haut nicht als störendes "Weißeln" (Bildung von weißen Flecken auf der Haut) beobachtet. Üblicherweise liegen die Partikelgrößen solcher Pigmente im Bereich unter 100 nm. In einer herkömmlichen Emulsion neigen die Partikel mehr oder weniger stark zur Zusammenlagerung zu Agglomeraten, welche bereits unter dem Lichtmikroskop zu erkennen sind. Solche Agglomeration ist ferner nicht mit dem Herstellungsprozeß einer entsprechenden Zubereitung abgeschlossen, sondern setzt sich während der Lagerung fort. Das "Weißeln" kann sich daher über einen längeren Zeitraum noch verstärken. Auch kann das Ausölen oder gar Brechen einer Emulsion mittel- oder langfristig Folge einer derartigen Agglomeration sein.

Ein weiterer Nachteil des Einsatzes anorganischer Pigmente in kosmetischen Formulierungen ist, daß solche Pigmente in den weitaus meisten Fällen zu starker Hauttrockenheit führen.

Dennoch bestand der Nachteil des Standes der Technik, daß in der Regel entweder nur vergleichsweise niedrige Lichtschutzfaktoren erreicht werden konnten, oder daß die Lichtschutzfilter nicht die genügende UV-Stabilität aufwiesen oder nicht genügende physiologische Verträglichkeit aufwiesen oder nicht genügend hohe Löslichkeit oder Dispergierbarkeit in kosmetischen oder dermatologischen Zubereitungen aufwiesen oder auch sonstige Inkompatibilitäten mit kosmetischen oder dermatologischen Zubereitungen oder mehrere Nachteile zugleich.

Häufige Erscheinungsformen kosmetischer oder dermatologischer Zubereitungen sind feindisperse Mehrphasensysteme, in welchen eine oder mehrere Fett- bzw. Ölphasen neben einer bzw. mehreren Wasserphasen vorliegen. Von diesen Systemen sind wiederum die eigentlichen Emulsionen die am weitesten verbreiteten.

In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Die Tröpfchendurchmesser der gewöhnlichen Emulsionen liegen im Bereich von ca 1 µm bis ca. 50 µm. Feinere "Makroemulsionen", deren Tröpfchendurchmesser im Bereich von ca. $10^{-1}$ µm bis ca. 1 µm liegen, sind, wiederum ohne färbende Zusätze, bläulichweißgefärbt und undurchsichtig.

Der Tröpfchendurchmesser von transparenten bzw. transluzenten Mikroemulsionen dagegen liegt im Bereich von etwa $10^{-2}$ µm bis etwa $10^{-1}$ µm. Solche Mikroemulsionen sind meist niedrigviskos. Die Viskosität vieler Mikroemulsionen vom O/W-Typ ist vergleichbar mit der des Wassers.

Nachteilig an vielen O/W-Emulsionen des Standes der Technik ist, daß stets ein hoher Gehalt an einem oder mehreren Emulgatoren eingesetzt werden muß, da die geringe Tröpfchengröße eine hohe Grenzfläche zwischen den Phasen bedingt, welche in der Regel durch Emulgatoren stabilisiert werden muß.

Wasserlösliche UV-Filtersubstanzen sind Elektrolyte, welche insbesondere O/W-Emulsionen destabilisieren. Um dieser Destabilisierung entgegenzuwirken, werden polyethoxylierte Emulgatoren eingesetzt. Diese haben aber oft dermatologische Nachteile, denn zwar ist die Verwendung der üblichen kosmetischen Emulgatoren unbedenklich. Dennoch können Emulgatoren, wie letztlich jede chemische Substanz, im Einzelfalle allergische oder auf Überempfindlichkeit des Anwenders beruhende Reaktionen hervorrufen. Außerdem weisen polyethoxylierte Emulgatoren in Kombination mit anderen Emulsionsbestandteilen eine verringerte Stabilität unter UV-Bestrahlung auf, was ihren Einsatz in Sonnenschutzmitteln einschränkt.

So ist bekannt, daß bestimmte Lichtdermatosen durch gewisse Emulgatoren, aber auch durch verschiedene Fette, und gleichzeitige Exposition von Sonnenlicht ausgelöst werden. Solche Lichtdermatosen werden auch "Mallorca-Akne"genannt. Eine Aufgabe der vorliegenden Erfindung war daher, Sonnenschutzprodukte zu entwickeln.

Eine weitere Aufgabe war es, Sonnenschutzmittel mit hoher Stabilität und mit hoher dermatologischer Sicherheit und Verträglichkeit zu entwickeln.

Noch eine weitere Aufgabe war es, durch geeignete Zubereitungen den Einsatzbereich wasserlöslicher, insbesondere sulfonierter UV-Filtersubstanzen zu erweitem.

So betrifft die vorliegende Erfindung als besondere Ausführungsformen kosmetische und dermatologische Lichtschutzzubereitungen, insbesondere hautpflegende kosmetische und dermatologische Lichtschutzzubereitungen.

Wenigstens einigen, wenn nicht allen diesen Nachteilen abzuhelfen, war also Aufgabe der vorliegenden Erfindung.

Es war indes überraschend und für den Fachmann nicht vorauszusehen, daß kosmetische oder pharmazeutische O/W-Emulsionen, welche

(a) eine oder mehrere grenzflächenaktiven Substanzen, gewählt aus der Gruppe der Glucosederivate, welche sich durch die Strukturformel

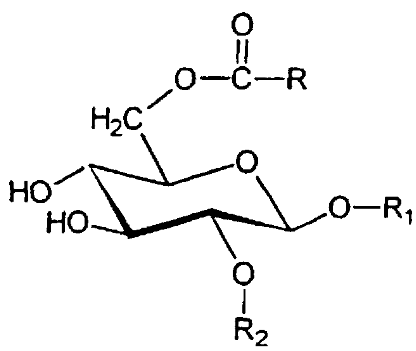

auszeichnen, wobei R einen verzweigten oder unverzweigten Alkylrest mit 1 bis 24 Kohlenstoffatomen darstellt, wobei $R_1$ entweder ein Wasserstoffatom oder einen verzweigten oder unverzweigten Alkylrest mit 1 bis 24 Kohlenstoffatomen darstellt und wobei $R_2$ entweder ein Wasserstoffatom oder einen verzweigten oder unverzweigten Acylrest mit 1 bis 24 Kohlenstoffatomen darstellt,

(b) und einen oder mehrere wasserlösliche, kosmetisch oder pharmazeutisch akzeptable UV-Filtersubstanzen,

(c) eine Wasserphase, welche gegebenenfalls übliche, darin lösliche und/oder dispergierbare Substanzen, darunter insbesondere weitere kosmetisch oder pharmazeutisch akzeptable UV-Filtersubstanzen, enthält,

(d) eine Ölphase, welche gegebenenfalls übliche, darin lösliche und/oder dispergierbare Substanzen, darunter insbesondere weitere kosmetisch oder pharmazeutisch akzeptable UV-Filtersubstanzen, enthält, enthalten und welche

(e) im wesentlichen frei von polyethoxylierten Emulgatoren sind, den Nachteilen des Standes der Technik abhelfen.

Insbesondere stellt aber auch die Verwendung von

(a) einer oder mehrerer grenzflächenaktiven Substanzen, gewählt aus der Gruppe der Glucosederivate, welche sich durch die Strukturformel

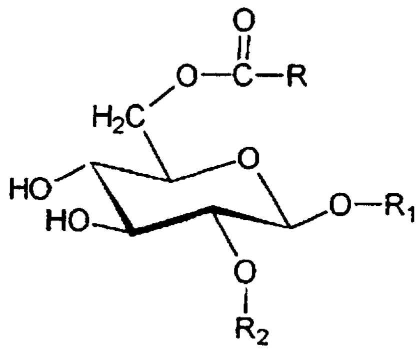

auszeichnen, wobei R einen verzweigten oder unverzweigten Alkylrest mit 1 bis 24 Kohlenstoffatomen darstellt, wobei $R_1$ entweder ein Wasserstoffatom oder einen verzweigten oder unverzweigten Alkylrest mit 1 bis 24 Kohlenstoffatomen darstellt und wobei $R_2$ entweder ein Wasserstoffatom oder einen verzweigten oder unverzweigten Acylrest mit 1 bis 24 Kohlenstoffatomen darstellt,
zur Stabilisierung kosmetischer oder pharmazeutischer O/W-EMulsionen, welche einen wirksamen Gehalt an einer oder mehreren wasserlöslichen, kosmetisch oder pharmazeutisch akzeptablen UV-Filtersubstanzen aufweisen, eine vorteilhafte Verkörperung der vorliegenden Erfindung dar, insbesondere, in O/W-Emulsionen, welche
(c) eine Wasserphase, welche gegebenenfalls übliche, darin lösliche und/oder dispergierbare Substanzen, darunter insbesondere weitere kosmetisch oder pharmazeutisch akzeptable UV-Filtersubstanzen, enthalten, und
(d) eine Ölphase, welche gegebenenfalls übliche, darin lösliche und/oder dispergierbare Substanzen, darunter insbesondere weitere kosmetisch oder pharmazeutisch akzeptable UV-Filtersubstanzen, enthalten,
enthalten, und welche
(e) im wesentlichen frei von polyethoxylierten Emulgatoren sind.

Vorteilhaft wird R gewählt aus der Gruppe der unverzweigten Alkylreste, wobei der Myristylrest, der Palmitylrest, der Stearylrest und der Eicosylrest bevorzugt werden.
$R_1$ kann vorteilhaft ein Wasserstoffatom darstellen, wird aber bevorzugt aus der Gruppe Methyl-, Ethyl-, Propyl- und Isopropyl- gewählt.
$R_2$ kann vorteilhaft ein Wasserstoffatom darstellen, kann aber ebenfalls vorteilhaft aus der Gruppe Myristoyl-, Palmitoyl-, Stearoyl- und Eicosoyl- gewählt werden.

Besonders vorteilhaft wird als grenzflächenaktive Substanzen aus der Gruppe der Glucosederivate das Methylglucosesesquistearat gewählt, welches zu etwa gleichen Teilen aus den Substanzen

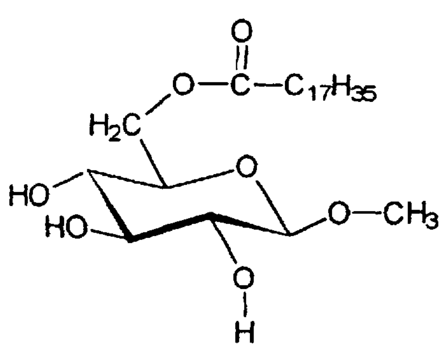

und

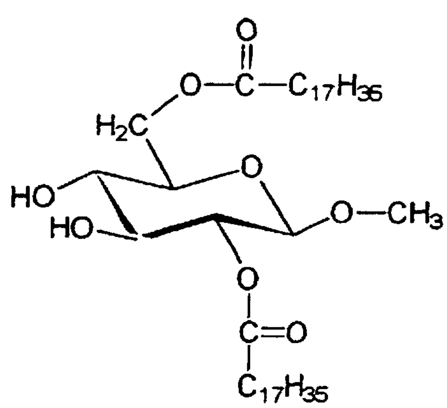

besteht. Solche Mischungen sind im Handel beispielsweise unter der Warenbezeichnung Tego® Care PS von der Gesellschaft Th.Goldschmidt KG erhältlich.

Erfindungsgemäß können diese grenzflächenaktiven Substanzen in Konzentrationen von 0,005 bis 50 Gew. %, bezogen auf das Gesamtgewicht der Zubereitungen, vorliegen. Dabei werden Konzentrationen von 0.5 - 10 Gew.-%, insbesondere 1,0 - 7 Gew.-%, bevorzugt.

Vorteilhafte wasserlösliche UV-Filtersubstanzen im Sinne der vorliegenden Erfindung sind insbesondere solche, die an ihrem Molekülgerüst eine oder mehrere Sulfonsäuregruppen bzw. Sulfonatgruppen tragen. Bevorzugt sind insbesondere :

Die 2-Phenylbenzimidazol-5-sulfonsäure und ihre Salze, beispielsweise das Natrium-, Kalium- oder ihr Triethanolammonium-Salz

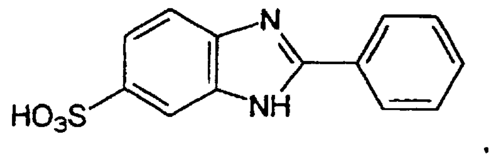

Sulfonsäure-Derivate von Benzophenonen, vorzugsweise 2-Hydroxy-4-methoxybenzophenon-5-sulfonsäure und ihre Salze, beispielsweise das entsprechende Natrium-, Kalium- oder Triethanolammonium-Salz:

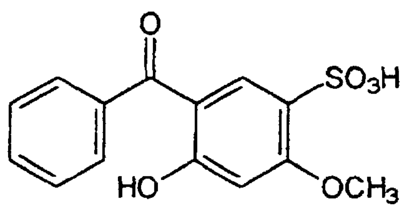

Sulfonsäure-Derivate des 3-Benzylidencamphers, wie z.B. 4-(2-Oxo-3-bornylidenmethyl)benzolsulfonsäure und ihre Salze, beispielsweise das entsprechende Natrium-, Kalium- oder Triethanolammonium-Salz:

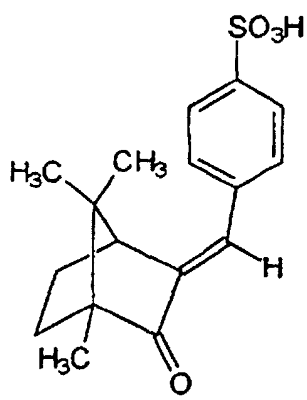

Die 2-Methyl-5-(2-oxo-3-bornylidenmethyl)benzolsulfonsäure und ihre Salze, beispielsweise das entsprechende Natrium-, Kalium- oder Triethanolammonium-Salz:

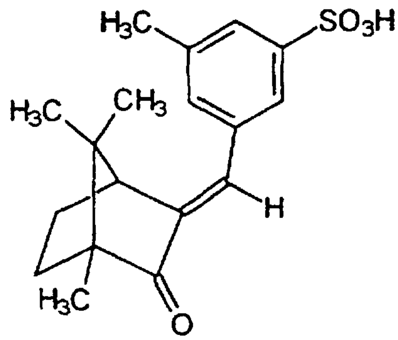

Das 1,4-di(2-oxo-10-Sulfo-3-bornylidenmethyl-)Benzol und dessen Salze (die entprehenden 10-Sulfato-verbindungen, beispielsweise das entsprechende Natrium-, Kalium- oder Triethanolammonium-Salz), auch als Benzol-1,4-di(2-oxo-3-bomylidenmethyl-10-Sulfonsäure bezeichnet:

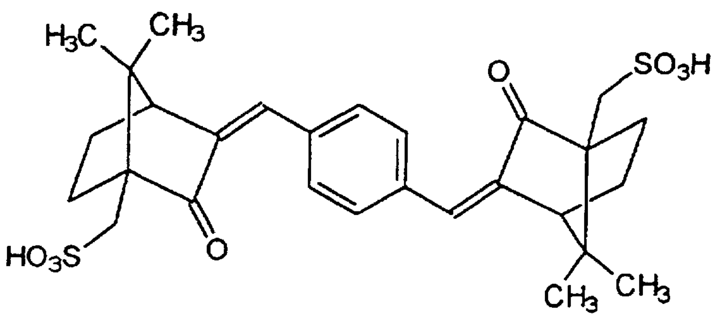

Die Phenylen-1,4-bis-(2-benzimidazyl)-3,3'-5,5'-tetrasulfonsäure:

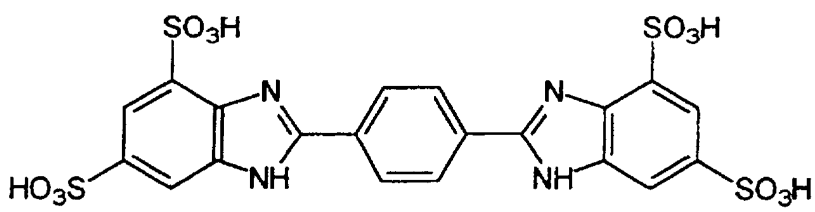

und ihre Salze, beispielsweise das entsprechende Natrium-, Kalium- oder Triethanolammonium-Salz, insbesondere das Phenylen-1,4-bis-(2-benzimidazyl)-3,3'-5,5'-tetrasulfonsäure-bis-natriumsalz:

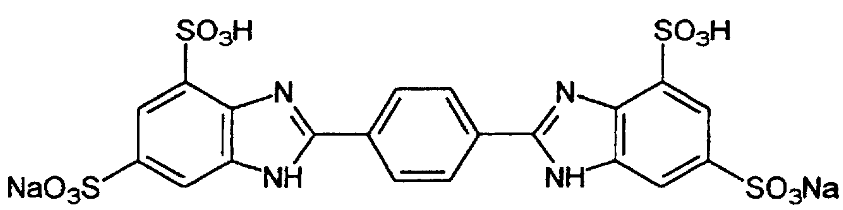

Solche Zubereitungen helfen den geschilderten Nachteilen des Standes der Technik in überraschender Weise ab. Erfindungsgemäß sind höhere Lichtschutzfaktoren erreichbar als der Stand der Technik dies hätte annehmen lassen.

In den erfindungsgemäßen kosmetischen oder dermatologischen Zubereitungen, haben ferner auch die schwererlöslichen Komponenten eine bessere Löslichkeit als in den Zubereitungen des Standes der Technik, auch dann, wenn mehrere solcher Komponenten vorliegen.

Erfindungsgemäß kann ferner die Agglomeration eventuell vorhandener anorganischer Pigmentpartikel (welche natürlich dispergiert, und nicht gelöst, vorliegen) mit den Folgen "Weißeln", Ausölen, Brechen der Emulsion verhindert werden, auch dann, wenn zusätzlich ein oder mehrere schwererlösliche Komponenten vorliegen.

Weiterhin sind erfindungsgemäß Lichtschutzzubereitungen erhältlich, welche höhere Stabilität, insbesondere Stabilität gegen Zersetzung unter dem Einfluß von Licht, ganz besonders UV-Licht, aufweisen, als der Stand der Technik hätte erwarten lassen. Insbesondere die Stabilität von 4-(tert.-Butyl)-4'-methoxydibenzoylmethan wird drastisch erhöht.

Weiterhin sind erfindungsgemäß besonders gut hautverträgliche Zubereitungen erhältlich, wobei wertvolle Inhaltsstoffe besonders gut auf der Haut verteilt werden können.

Es ist erfindungsgemäß möglich die Einsatzmengen von 4,4',4"-(1,3,5-Triazin-2,4,6-triyltriimino)-tris-benzoesäure-tris(2-ethylhexylester), aber auch der anderen unter Normalbedingungen als Feststoff vorliegenden Lichschutzfiltersubstanzen, in kosmetischen oder dermatologischen Zubereitungen gegenüber dem Stande der Technik wesentlich zu erhöhen.

Ferner war erstaunlich, daß erfindungsgemäß eine Stabilisierung von Lösungen des 4.4',4"-(1,3,5-Triazin-2,4,6-triyltriimino)-tris-benzoesäure-tris(2-ethylhexylesters) bewirkt wird, da die letztere Substanz nicht nur schlechte Löslichkeit aufweist, sondern auch aus ihrer Lösung leicht wieder auskristallisiert.

Die Gesamtmenge an der oder den wasserlöslichen UV-Filtersubstanzen in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Phenylbenzimidazol-5-sulfonsäure (sofern es diese Substanz ist, welche als wasserlösliche UV-Filtersubstanz im Sinne der vorliegenden Erfindung eingesetzt werden soll) bzw. deren Salzen in den fertigen kosmetischen oder dermatologischen Zubereitungen wird, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Hydroxy-4-methoxybenzophenon-5-sulfonsäure (sofern es diese Substanz ist, welche als wasserlösliche UV-Filtersubstanz im Sinne der vorliegenden Erfindung eingesetzt werden soll) bzw. deren Salzen in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4-(2-Oxo-3-bornylidenmethyl)benzolsulfonsäure (sofern es diese Substanz ist, welche als wasserlösliche UV-Filtersubstanz im Sinne der vorliegenden Erfindung eingesetzt werden soll) bzw. deren Salzen in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Methyl-5-(2-oxo-3-bornylidenmethyl)benzolsulfonsäure (sofern es diese Substanz ist, welche als wasserlösliche UV-Filtersubstanze im Sinne der vorliegenden Erfindung eingesetzt werden soll) bzw. deren Salzen in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an Benzol-1,4-di(2-oxo-3-bornylidenmethyl-10-Sulfonsäure (sofern es diese Substanz ist, welche als wasserlösliche UV-Filtersubstanze im Sinne der vorliegenden Erfindung eingesetzt werden soll) bzw. deren Salzen in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an Phenylen-1,4-bis-(2-benzimidazyl)-3,3'-5,5'-tetrasulfonsäure (sofern es diese Substanz ist, welche als sulfonierte UV-Filtersubstanz im Sinne der vorliegenden Erfindung eingesetzt werden soll) bzw. deren Salzen in den fertigen kosmetischen oder dermatologischen Zubereitungen wird vorteilhaft aus dem Bereich von 0,1 10,0 Gew.-%, bevorzugt 0,5 - 6,0 Gew.-% gewählt, bezogen auf das Gesamtgewicht der Zubereitungen.

Die Gesamtmenge an 4,4',4"-(1,3,5-Triazin-2,4,6-triyltriimino)-tris-benzoesäure-tris-(2-ethylhexylester) (als an sich fakultativ einzusetzender zusätzlicher UV-Filtersubstanz) in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4-(tert.-Butyl)-4'-methoxydibenzoylmethan (als an sich fakultativ einzusetzender zusätzlicher UV-Filtersubstanz) in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4-Methylbenzylidencampher (als an sich fakultativ einzusetzender zusätzlicher UV-Filtersubstanz) in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Ethylhexyl-p-methoxy-cinnamat (als an sich fakultativ einzusetzender zusätzlicher UV-Filtersubstanz) in den fertigen kosmetischen oder dermatologischen Zubereitungen wird vorteilhaft aus dem Bereich von 0,1 - 15,0 Gew.-%, bevorzugt 0,5 - 7,5 Gew.-% gewählt, bezogen auf das Gesamtgewicht der Zubereitungen.

Ferner kann gegebenenfalls von Vorteil sein, die erfindungsgemäßen Wirkstoffkombinationen mit weiteren UVA- und/oder UVB-Filtern zu kombinieren, beispielsweise bestimmten Salicylsäurederivaten wie

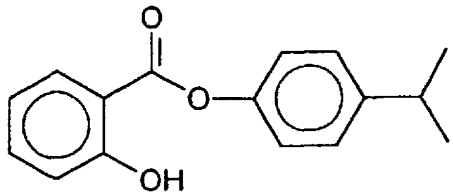

(4-Isopropylbenzylsalicylat),

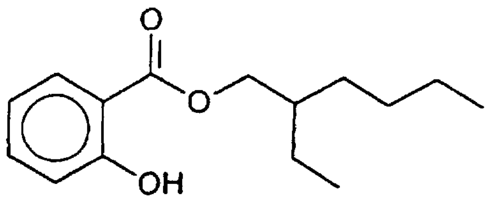

(2-Ethylhexylsalicylat, Octylsalicylat),

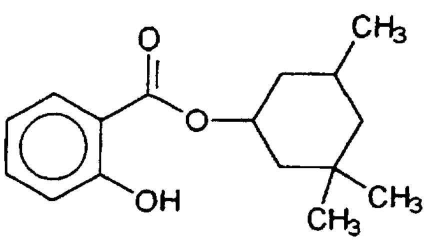

(Homomenthylsalicylat).

Die Gesamtmenge an einem oder mehreren Salicylsäurederivaten in den fertigen kosmetischen oder dermatologischen Zubereitungen wird vorteilhaft aus dem Bereich von 0,1 - 15,0 Gew.-%, bevorzugt 0,5 - 8,0 Gew.-% gewählt, bezogen auf das Gesamtgewicht der Zubereitungen. Wenn Ethylhexylsalicylat gewählt wird, ist es von Vorteil, dessen Gesamtmenge aus dem Bereich von 0,1 - 5,0 Gew.-%, bevorzugt 0,5 - 2,5 Gew.-% zu wählen. Wenn Homomenthylsalicylat gewählt wird, ist es von Vorteil, dessen Gesamtmenge aus dem Bereich von 0,1 - 10,0 Gew.-%, bevorzugt 0,5 - 5,0 Gew.-% zu wählen.

Die Gesamtmenge an Ethylhexyl-2-cyano-3,3-diphenylacrylat (als an sich fakultativ einzusetzender zusätzlicher UV-Filtersubstanz) in den fertigen kosmetischen oder dermatologischen Zubereitungen wird vorteilhaft aus dem Bereich von 0,1 - 15,0 Gew.-%, bevorzugt 0,5 - 10,0 Gew.-% gewählt, bezogen auf das Gesamtgewicht der Zubereitungen.

Es kann auch von Vorteil sein, die erfindungsgemäßen Kombinationen mit UVA-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Es ist erfindungsgemäß vorteilhaft, außer den erfindungsgemäßen Kombinationen weitere UVA-Filter und/oder UVB-Filter einzusetzen, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Vorteilhafte öllösliche UVB-Filtersubstanzen sind z.B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3.5-triazin.

Die Liste der genannten UVB-Filter, die in Kombination mit den erfindungsgemäßen Wirkstoffkombinationen verwendet werden können, soll selbstverständlich nicht limitierend sein.

Ferner ist vorteilhaft, die erfindungsgemäßen Wirkstoffkombinationen mit weiteren UVA- und/oder UVB-Filtern zu kombinieren.

Erfindungsgemäße kosmetische und dermatologische Zubereitungen enthalten außerdem vorteilhaft, aber nicht zwingend, anorganische Pigmente auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$, Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Die anorganischen Pigmente liegen bevorzugt in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

$$n\ TiO_2 + m\ (RO)_3Si\text{-}R' \rightarrow n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen T 805 von der Firma Degussa oder MT 100 T von der Firma Tayca oder M 160 von der Frima Kemira erhältlich.

Als wasserdispergierbare anorganische Mikropigmente könnnen beispielsweise solche Produkte gewählt werden, welche unter der Handelsbezeichnung Tioveil® von der Firma Tioxide erhältlich sind.

Die Gesamtmenge an anorganischen Pigmenten, insbesondere hydrophoben anorganischen Mikropigmenten (als an sich fakultativ einzusetzender zusätzlicher Substanz) in den fertigen kosmetischen oder dermatologischen Zubereitungen wird vorteilhaft aus dem Bereich von 0,1 - 30 Gew.-%, bevorzugt 0,1 - 10,0, insbesondere 0,5 - 6,0 Gew.-% gewählt, bezogen auf das Gesamtgewicht der Zubereitungen.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen

Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Ölphase der erfindungsgemäßen Zubereitungen wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen.

Vorteilhaft wird die Ölphase gewählt aus der Gruppe 2-Ethylhexylisostearat, Octyldodecanol. Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind femer Mischungen aus Cyclomethicon und Isotridecylisononanoat, aus Cyclomethicon und 2-Ethylhexylisostearat.

Vorteilhaft beträgt der Gehalt an der Ölphase zwischen 1 und 50 Gew.-%, bezogen auf das Gesamtgewicht der Zubereitungen, bevorzugt 2.5 - 30 Gew.-%, insbesondere bevorzugt 5 - 15 Gew.-%.

Als Co-Emulgatoren können weiter vorteilhaft Substanzen gewählt werden, gewählt aus der Gruppe der Sorbitanester und der Glycerinester.

Die Glycerinester werden bevorzugt gewählt aus der Gruppe der grenzflächenaktiven Substanzen, gewählt aus der Gruppe der Glycerin-mono- bzw. -dicarbonsäure-monoester der allgemeinen Formel

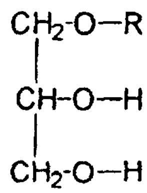

bzw.

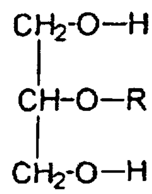

.

wobei R einen verzweigten oder unverzweigten Acylrest mit 6 - 24 Kohlenstoffatomen darstellt, bevorzugt Glycerinmonostearat.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Beispiel 1**

| | Gew.-% |
|---|---|
| Methylglucosesesquistearat | 3,00 |
| Caprylic/Capric Triglyceride | 5,00 |
| Octyldodecanol | 5,00 |
| Dicaprylylether | 2.00 |
| Phenylbenzimidazolsulfonsäure | 4,00 |
| Glycerin | 3,00 |
| Carbomer | 0,50 |
| Tocopherylacetat | 1,00 |
| NaOH | q.s. |
| Parfum, Konservierungsmittel | q.s. |

(fortgesetzt)

| | Gew.-% |
|---|---|
| Wasser | ad 100,00 |

**Beispiel 2**

| | Gew.-% |
|---|---|
| Methylglucosesesquistearat | 5,00 |
| Caprylic/Capric Triglyceride | 1,67 |
| Octyldodecanol | 1,67 |
| Dicaprylylether | 1,67 |
| Phenylbenzimidazolsulfonsäure | 4,00 |
| Butylenglycol | 3,00 |
| Tocopherylacetat | 1,00 |
| KOH | q.s. |
| Parfum, Konservierungsmittel | q.s. |
| Wasser | ad 100,00 |

**Beispiel 3**

| | Gew.-% |
|---|---|
| Methylglucosesesquistearat | 5,00 |
| Caprylic/Capric Triglyceride | 5,00 |
| Octyldodecanol | 5,00 |
| Dicaprylylether | 1,67 |
| Benzol-1,4-di(2-oxo-3-bornylidenmethyl-10-Sulfonsäure) | 4,00 |
| Glycerin | 3,00 |
| Tocopherylacetat | 1,00 |
| NaOH | q.s. |
| Parfum, Konservierungsmittel | q.s. |
| Wasser | ad 100,00 |

**Beispiel 4**

| | Gew.-% |
|---|---|
| Methylglucosesesquistearat | 5,00 |
| Caprylic/Capric Triglyceride | 1,67 |
| Octyldodecanol | 1,67 |
| Dicaprylylether | 1,67 |
| Phenylbenzimidazolsulfonsäure | 2,00 |
| Tris-[anilino-(p-carbo-2'-ethyl-1'-hexyloxy)]-triazin | 3,00 |
| 4-(tert.-Butyl)-4'-methoxydibenzoylmethan | 2,00 |
| Butylenglycol | 3,00 |
| Glycerinmonosterarat | 2,00 |
| 4-Methyl-Benzylidencampher | 2,00 |

(fortgesetzt)

| | Gew.-% |
|---|---|
| Tocopherylacetat | 1,00 |
| NaOH | q.s. |
| Parfum, Konservierungsmittel | q.s. |
| Wasser | ad 100,00 |

## Patentansprüche

**1.** Kosmetische oder pharmazeutische O/W-Emulsionen, welche

(a) eine oder mehrere grenzflächenaktiven Substanzen, gewählt aus der Gruppe der Glucosederivate, welche sich durch die Strukturformel

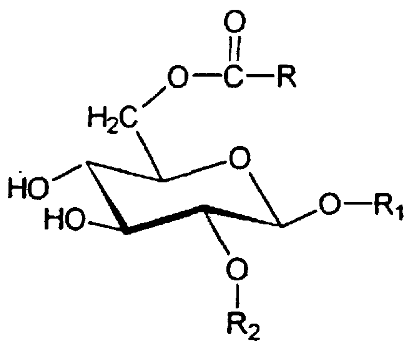

auszeichnen, wobei R einen verzweigten oder unverzweigten Alkylrest mit 1 bis 24 Kohlenstoffatomen darstellt, wobei $R_1$ entweder ein Wasserstoffatom oder einen verzweigten oder unverzweigten Alkylrest mit 1 bis 24 Kohlenstoffatomen darstellt und wobei $R_2$ entweder ein Wasserstoffatom oder einen verzweigten oder unverzweigten Acylrest mit 1 bis 24 Kohlenstoffatomen darstellt,
(b) und einen oder mehrere wasserlösliche, kosmetisch oder pharmazeutisch akzeptable UV-Filtersubstanzen,
(c) eine Wasserphase, welche gegebenenfalls übliche, darin lösliche und/oder dispergierbare Substanzen, darunter insbesondere weitere kosmetisch oder pharmazeutisch akzeptable UV-Filtersubstanzen, enthält,
(d) eine Ölphase, welche gegebenenfalls übliche, darin lösliche und/oder dispergierbare Substanzen, darunter insbesondere weitere kosmetisch oder pharmazeutisch akzeptable UV-Filtersubstanzen, enthält,
enthalten und welche
(e) im wesentlichen frei von polyethoxylierten Emulgatoren sind.

**2.** Verwendung von

(a) einer oder mehrerer grenzflächenaktiven Substanzen, gewählt aus der Gruppe der Glucosederivate, welche sich durch die Strukturformel

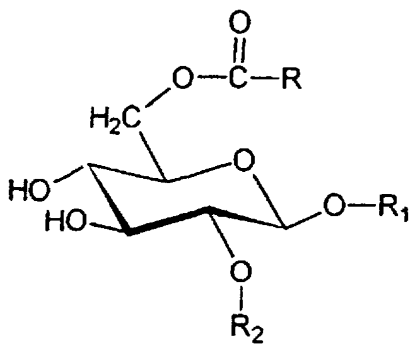

auszeichnen, wobei R einen verzweigten oder unverzweigten Alkylrest mit 1 bis 24 Kohlenstoffatomen darstellt, wobei $R_1$ entweder ein Wasserstoffatom oder einen verzweigten oder unverzweigten Alkylrest mit 1 bis 24 Kohlenstoffatomen darstellt und wobei $R_2$ entweder ein Wasserstoffatom oder einen verzweigten oder unverzweigten Acylrest mit 1 bis 24 Kohlenstoffatomen darstellt,

zur Stabilisierung kosmetischer oder pharmazeutischer O/W-EMulsionen, welche einen wirksamen Gehalt an einer oder mehreren wasserlöslichen, kosmetisch oder pharmazeutisch akzeptablen UV-Filtersubstanzen aufweisen.

**3.** Verwendung nach Anspruch 2 in O/W-Emulsionen, welche

(c) eine Wasserphase, welche gegebenenfalls übliche, darin lösliche und/oder dispergierbare Substanzen, darunter insbesondere weitere kosmetisch oder pharmazeutisch akzeptable UV-Filtersubstanzen, enthalten, und
(d) eine Ölphase, welche gegebenenfalls übliche, darin lösliche und/oder dispergierbare Substanzen, darunter insbesondere weitere kosmetisch oder pharmazeutisch akzeptable UV-Filtersubstanzen, enthalten, enthalten, und welche
(e) im wesentlichen frei von polyethoxylierten Emulgatoren sind.

**4.** O/W-Emulsionen nach Anspruch 1 oder Verwendung nach Anspruch 2, **dadurch gekennzeichnet, daß** in der Strukturformel des oder der grenzflächenaktiven Substanzen aus der Gruppe der Glucosederivate der Rest R gewählt wird aus der Gruppe Myristyl-, Palmityl-, Stearyl-, Eicosyl-, der Rest $R_1$ ein Wasserstoffatom darstellt oder aus der Gruppe Methyl-, Ethyl-, Propyl- und Isopropyl- gewählt wird, und/oder der Rest $R_2$ ein Wasserstoffatom darstellt oder aus der Gruppe Myristoyl- , Palmitoyl-, Stearoyl-, Eicosoyl- gewählt wird.

**5.** O/W-Emulsionen nach Anspruch 1 oder Verwendung nach Anspruch 2, **dadurch gekennzeichnet, daß** als grenzflächenaktive Substanz aus der Gruppe der Glucosederivate das Methylglucosesesquistearat gewählt wird.

**6.** O/W-Emulsionen nach Anspruch 1 oder Verwendung nach Anspruch 2, **dadurch gekennzeichnet, daß** die wasserlösliche, kosmetisch oder pharmazeutisch akzeptabler UV-Filtersubstanz oder -substanzen gewählt werden aus der Gruppe der UV-Filtersubstanzen, die an ihrem Molekülgerüst eine oder mehrere Sulfonsäuregruppen bzw. Sulfonatgruppen tragen.

**7.** O/W-Emulsionen nach Anspruch 1 oder Verwendung nach Anspruch 2, **dadurch gekennzeichnet, daß** die wasserlösliche, kosmetisch oder pharmazeutisch akzeptabler UV-Filtersubstanz oder -substanzen gewählt werden aus der Gruppe 2-Phenylbenzimidazol-5-sulfonsäure und ihre Salze, der Sulfonsäure-Derivate von Benzophenonen, vorzugsweise 2-Hydroxy-4-methoxybenzophenon-5-sulfonsäure und ihre Salze, der Sulfonsäure-Derivate des 3-Benzylidencamphers, wie z.B. 4-(2-Oxo-3-bornylidenmethyl)benzolsulfonsäure und ihre Salze, beispielsweise das entsprechende Natrium-, Kalium- oder Triethanolammonium-Salz 2-Methyl-5-(2-oxo-3-bornylidenmethyl)benzolsulfonsäure und ihre Salze, beispielsweise das entsprechende Natrium-, Kalium- oder Triethanolammonium-Salz, 1,4-di(2-oxo-10-Sulfo-3-bornylidenmethyl)Benzol und dessen Salze.

## Claims

**1.** Cosmetic or pharmaceutical O/W emulsions which contain

(a) one or more surface-active substances selected from the group consisting of glucose derivatives of the structural formula

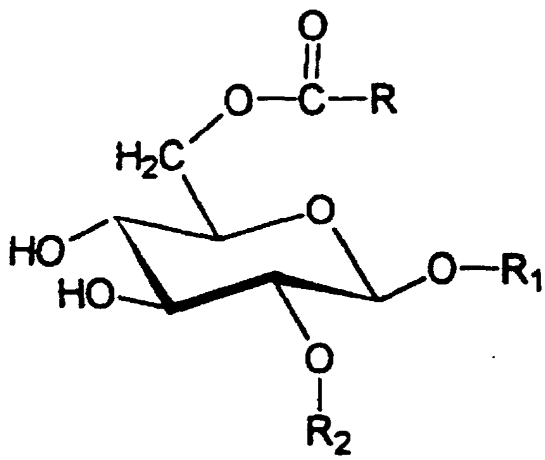

in which R is a branched or unbranched alkyl radical having 1 to 24 carbon atoms, $R_1$ is either a hydrogen atom or a branched or unbranched alkyl radical having 1 to 24 carbon atoms and $R_2$ is either a hydrogen atom or a branched or unbranched acyl radical having 1 to 24 carbon atoms,

(b) one or more water-soluble, cosmetically or pharmaceutically acceptable UV filters,

(c) an aqueous phase which may contain conventional substances soluble and/or dispersible therein, including especially other cosmetically or pharmaceutically acceptable UV filters, and

(d) an oil phase which may contain conventional substances soluble and/or dispersible therein, including especially other cosmetically or pharmaceutically acceptable UV filters,

and which are

(e) essentially free of polyethoxylated emulsifiers.

**2.** Use of

(a) one or more surface-active substances selected from the group consisting of glucose derivatives of the structural formula

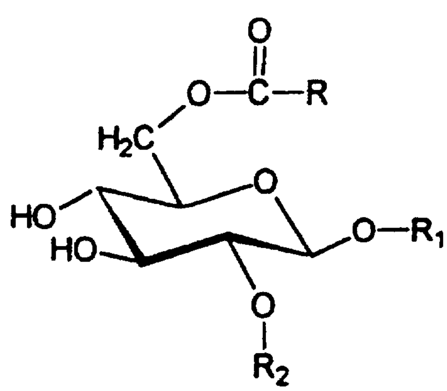

in which R is a branched or unbranched alkyl radical having 1 to 24 carbon atoms, $R_1$ is either a hydrogen atom or a branched or unbranched alkyl radical having 1 to 24 carbon atoms and $R_2$ is either a hydrogen atom or a branched or unbranched acyl radical having 1 to 24 carbon atoms,

for the stabilization of cosmetic or pharmaceutical O/W emulsions which have an effective content of one or more water-soluble, cosmetically or pharmaceutically acceptable UV filters.

**3.** Use according to Claim 2 in O/W emulsions which contain

(c) an aqueous phase which may contain conventional substances soluble and/or dispersible therein, including especially other cosmetically or pharmaceutically acceptable UV filters, and
(d) an oil phase which may contain conventional substances soluble and/or dispersible therein, including especially other cosmetically or pharmaceutically acceptable UV filters,
and which are
(e) essentially free of polyethoxylated emulsifiers.

**4.** O/W emulsions according to Claim 1 or use according to Claim 2, **characterized in that**, in the structural formula of the surface-active substance or substances from the group consisting of glucose derivatives, the radical R is selected from the group consisting of myristyl, palmityl, stearyl and eicosyl, the radical $R_1$ is a hydrogen atom or is selected from the group consisting of methyl, ethyl, propyl and isopropyl, and/or the radical $R_2$ is a hydrogen atom or is selected from the group consisting of myristoyl, palmitoyl, stearoyl and eicosoyl.

**5.** O/W emulsions according to Claim 1 or use according to Claim 2, **characterized in that** methyl glucose sesquistearate is chosen as the surface-active substance from the group consisting of glucose derivatives.

**6.** O/W emulsions according to Claim 1 or use according to Claim 2, **characterized in that** the water-soluble, cosmetically or pharmaceutically acceptable UV filter or filters are selected from the group consisting of UV filters which carry one or more sulphonic acid groups or sulphonate groups on their molecular backbone.

**7.** O/W emulsions according to Claim 1 or use according to Claim 2, **characterized in that** the water-soluble, cosmetically or pharmaceutically acceptable UV filter or filters are selected from the group consisting of 2-phenylbenzimidazole-5-sulphonic acid and its salts, sulphonic acid derivatives of benzophenones, preferably 2-hydroxy-4-methoxybenzophenone-5-sulphonic acid and its salts, sulphonic acid derivatives of 3-benzylidenecamphor, e. g. 4-(2-oxo-3-bomylidenemethyl)-benzenesulphonic acid and its salts, for example the corresponding sodium, potassium or triethanolammonium salt, 2-methyl-5-(2-oxo-3-bornylidenemethyl)benzenesulphonic acid and its salts, for example the corresponding sodium, potassium or triethanolammonium salt, and 1,4-di(2-oxo-10-sulpho-3-bornylidenemethyl)benzene and its salts.

## Revendications

**1.** Emulsions H/E cosmétiques ou pharmaceutiques, comprenant

(a) une ou plusieurs substances tensioactives, choisies parmi le groupe constitué des dérivés du glucose, qui se distinguent par la formule structurale

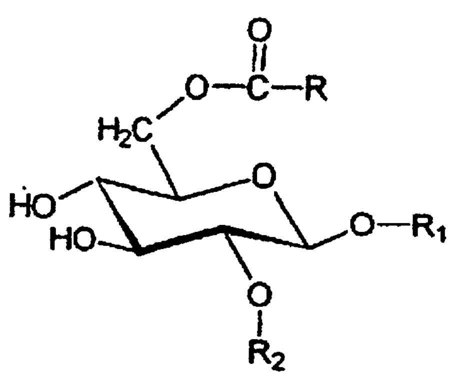

dans laquelle R représente un radical alkyle ramifié ou non ramifié ayant de 1 à 24 atomes de carbone, $R_1$ représente soit un atome d'hydrogène soit un radical alkyle ramifié ou non ramifié ayant de 1 à 24 atomes de carbone, et $R_2$ représente soit un atome d'hydrogène, soit un radical acyle ramifié ou non ramifié ayant de 1 à 24 atomes de carbone,
(b) et une ou plusieurs substances filtrant les UV hydrosolubles, cosmétiquement ou pharmaceutiquement

acceptables,
(c) une phase aqueuse comprenant éventuellement des substances habituelles qui y sont solubles et/ou dispersables, parmi lesquelles en particulier d'autres substances filtrant les UV cosmétiquement ou pharmaceutiquement acceptables,
(d) une phase huileuse comprenant éventuellement des substances habituelles qui y sont solubles et/ou dispersables, parmi lesquelles en particulier d'autres substances filtrant les UV cosmétiquement ou pharmaceutiquement acceptables,
et qui sont
(e) essentiellement exemptes d'émulsifiants polyéthoxylés.

**2.** Utilisation de

(a) une ou plusieurs substances tensioactives, choisies parmi le groupe constitué des dérivés du glucose, qui se distinguent par la formule structurale

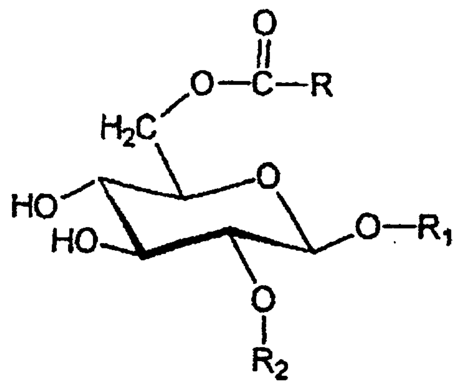

dans laquelle R représente un radical alkyle ramifié ou non ramifié ayant de 1 à 24 atomes de carbone, $R_1$ représente soit un atome d'hydrogène soit un radical alkyle ramifié ou non ramifié ayant de 1 à 24 atomes de carbone, et $R_2$ représente soit un atome d'hydrogène, soit un radical acyle ramifié ou non ramifié ayant de 1 à 24 atomes de carbone,

pour la stabilisation d'émulsions H/E cosmétiques ou pharmaceutiques présentant une teneur efficace en une ou plusieurs substances filtrant les UV hydrosolubles, cosmétiquement ou pharmaceutiquement acceptables.

**3.** Utilisation selon la revendication 2 dans des émulsions H/E, comprenant

(c) une phase aqueuse comprenant éventuellement des substances habituelles qui y sont solubles et/ou dispersables, parmi lesquelles en particulier d'autres substances filtrant les UV cosmétiquement ou pharmaceutiquement acceptables et
(d) une phase huileuse comprenant éventuellement des substances habituelles qui y sont solubles et/ou dispersables, parmi lesquelles en particulier d'autres substances filtrant les UV cosmétiquement ou pharmaceutiquement acceptables,
et qui sont
(e) essentiellement exemptes d'émulsifiants polyéthoxylés.

**4.** Emulsions H/E selon la revendication 1 ou utilisation selon la revendication 2, **caractérisées en ce que** dans la formule structurale de la ou des substance (s) tensioactive(s) parmi le groupe constitué des dérivés du glucose, le radical R est choisi parmi le groupe constitué d'un myristyle, d'un palmityle, d'un stéaryle et d'un eicosyle, le radical $R_1$ représente un atome d'hydrogène ou est choisi parmi le groupe constitué d'un méthyle, d'un éthyle, d'un propyle et d'un isopropyle, et/ou le radical $R_2$ représente un atome d'hydrogène ou est choisi parmi le groupe constitué d'un myristoyle, d'un palmitoyle, d'un stéaroyle et d'un eicosoyle.

**5.** Emulsions H/E selon la revendication 1 ou utilisation selon la revendication 2, **caractérisées en ce que** le méthylglucose-sesquistéarate est choisi en tant que substance tensioactive parmi le groupe constitué des dérivés

du glucose.

6. Emulsions H/E selon la revendication 1 ou utilisation selon la revendication 2, **caractérisées en ce que** la substance ou les substances filtrant les UV hydrosoluble(s), cosmétiquement ou pharmaceutiquement acceptable(s) est (sont) choisie(s) parmi le groupe constitué des substances filtrant les UV, portant un ou plusieurs groupes acide sulfonique ou groupes sulfonate sur leur squelette moléculaire.

7. Emulsions H/E selon la revendication 1 ou utilisation selon la revendication 2, **caractérisées en ce que** la substance ou les substances filtrant les UV hydrosoluble(s), cosmétiquement ou pharmaceutiquement acceptable(s) est (sont) choisie(s) parmi le groupe constitué de l'acide 2-phénylbenzimidazole-5-sulfonique et de ses sels, des dérivés d'acide sulfonique de benzophénones, de préférence de l'acide 2-hydroxy-4-méthoxybenzophénone-5-sulfonique et de ses sels, des dérivés d'acide sulfonique du 3-benzylidènecamphre, comme par exemple de l'acide 4-(2-oxo-3-bornylidèneméthyl)benzènesulfonique et de ses sels, par exemple le sel de sodium, de potassium ou de triéthanolammonium correspondant, de l'acide 2-méthyl-5-(2-oxo-3-bornylidèneméthyl)benzènesulfonique et de ses sels, par exemple le sel de sodium, de potassium ou de triéthanolammonium correspondant, et du 1,4-di(2-oxo-10-sulfo-3-bornylidèneméthyl)benzène et de ses sels.